# FASCICULE DE BREVET EUROPEEN

(11) **EP 0 746 364 B1**
(45) Date de publication et mention de la délivrance du brevet: **20.05.1998**
(21) Numéro de dépôt: 95909829.4
(22) Date de dépôt: 15.02.1995
(51) Int. Cl.: A61M 5/32

(54) **DISPOSITIF DE SECURITE POUR SERINGUE A USAGE DENTAIRE**
SICHERHEITSVORRICHTUNG FÜR ZAHNÄRZTLICHE SPRITZE
SECURITY DEVICE FOR A SYRINGE USED IN DENTISTRY

(30) Priorité: 15.02.1994 FR 9401693
(43) Date de publication de la demande: 11.12.1996
(73) Titulaire: Jouvin, Jean-Luc, F-72000 Le Mans (FR)
(72) Inventeur: Jouvin, Jean-Luc, F-72000 Le Mans (FR)
(74) Mandataire: Pernez, Helga
(86) Numéro de dépôt international: FR9500176
(87) Numéro de publication internationale: WO9521646

(56) Documents cités:
- EP-A- 0 513 869
- FR-A- 2 439 021
- FR-A- 2 684 303
- US-A- 4 772 272
- US-A- 4 927 018
- US-A- 5 267 977

## Description

La présente invention concerne une seringue d'injection dentaire de sécurité, propre à limiter le risque de contamination résultant de piqûres intempestives.

Il est connu de conditionner les seringues sous un étui protecteur, et de livrer les aiguilles avec un capuchon permettant de protéger l'aiguille après usage.

Il est toutefois apparu que le recapuchonage peut lui-même être à l'origine de piqûres intempestives, la mise en place d'un capuchon présentant un canal de faible section sur une aiguille fine nécessitant une certaine habileté.

Par ailleurs, le recapuchonage n'empêche pas la réutilisation, volontaire ou non, de l'aiguille.

On connaît également dans l'état de la technique diverses seringues de sécurité, dont certaines permettent de garantir l'usage unique de l'aiguille. Outre la sécurité apportée par de telles seringues, de tels dispositifs rassurent le patient sur le fait que l'aiguille employée par son dentiste présente toutes les garanties de stérilité et que la seringue n'a jamais été employée sur un autre patient.

A titre d'exemple, le brevet français FR2669540 divulgue un dispositif protecteur pour seringue d'injection comportant un corps cylindrique fermé à l'une de ses extrémités par une paroi transversale dans laquelle l'aiguille est implantée. L'extrémité du corps cylindrique est équipée d'une glissière pour un protecteur d'aiguille comportant une première partie tubulaire de grand diamètre susceptible de glisser le long de la glissière entre deux positions éloignées axialement l'une de l'autre et une partie de petit diamètre prolongeant longitudinalement la partie de grand diamètre, formant gaine mobile de protection de l'aiguille. Des éléments de guidage et d'indexation sont prévus entre la glissière et la partie de gros diamètre du protecteur pour n'autoriser qu'un seul dégainage et regainage successif de l'aiguille.

La fabrication d'un tel dispositif est relativement complexe et coûteuse, et une telle seringue ne répond donc pas pleinement aux besoins du marché.

Une autre seringue de sécurité est décrite dans la demande de brevet WO93/14800. Elle comprend un logement tubulaire allongé dans lequel se loge un protège-aiguille allongé qui est relié de manière télescopique au logement. Un cylindre de seringue est monté coaxialement dans le protège-aiguille et comprend sur une de ses extrémités une aiguille et sur son extrémité opposée une plaque terminale. La plaque terminale s'encliquette dans une rainure formée dans une bride transversale du logement et attache ainsi de manière fixe le cylindre de seringue au logement. Une plaque d'étanchéité recouvre la partie terminale verrouillée en pénétrant dans une autre rainure formée dans la bride. Une ou plusieurs pattes de verrouillage souples pouvant être abaissées sont portées sur le protège-aiguille et s'étendent dans des ouvertures alignées correspondantes formées dans le logement pour bloquer le protège-aiguille en position déployée dans laquelle il recouvre l'aiguille. Lorsqu'on appuie sur les pattes de verrouillage, et qu'on exerce une pression dirigée vers l'intérieur sur l'extrémité avant du porte-aiguille, ce dernier peut se déplacer et s'opposer à la tension d'un ressort à boudin situé dans le boîtier et découvrir et libérer ainsi l'aiguille pour permettre d'extraire le médicament de la carpule de médicament ou d'effectuer une injection.

L'utilisation d'un tel dispositif n'est pas très ergonomique, et nécessite une certaine habileté du praticien. Par ailleurs, le dispositif divulgué nécessite la fabrication et l'assemblage d'un nombre élevé de pièces, notamment d'un ressort, ce qui rend son prix de revient élevé.

Le brevet européen EP250104 décrit une seringue présentant un corps extérieur protégeant l'aiguille avant usage. Il s'agit d'une seringue à usage unique dont le prix de revient est relativement élevé.

On connaît également dans l'état de la technique le brevet français FR2684303 décrivant un dispositif de sécurité pour seringue conforme au préambule de la revendication principale. Ce dispositif présente une fente longitudinale et un ergot formant une partie saillante.

Un tel dispositif présente des difficultés d'assemblage du porte-aiguille et du manchon.

L'objet de la présente invention est de proposer un dispositif de sécurité efficace et fiable, facilitant l'assemblage du porte-aiguille et du manchon.

Elle vise également à proposer un dispositif permettant de compléter une seringue de type standard, utilisable indifféremment avec des aiguilles conventionnelles ou avec le dispositif de protection selon l'invention. Le praticien peut ainsi investir dans une seringue de qualité qu'il équipera du type d'aiguille adapté à une situation particulière, plutôt que de choisir pour chaque intervention parmi des seringues de types différents.

L'invention a plus particulièrement pour objet un dispositif de sécurité pour seringue à usage dentaire caractérisé en ce que le manchon tubulaire présente une languette déformable élastiquement s'étendant au repos en travers d'une fente longitudinale prévue sur le manchon tubulaire, et dont l'extrémité est dirigée vers l'avant du manchon.

Le dispositif selon l'invention est constitué principalement de deux composantes fabriquées par moulage et nécessitant que des opérations d'assemblage facilement industrialisables.

Pour la bonne compréhension de la description, on emploiera le terme d'avancée, de distale ou d'extrémité avant l'extrémité du manchon la plus éloignée du col de la seringue lorsque les deux éléments sont assemblés, l'extrémité opposée du manchon tubulaire étant désignée par l'extrémité arrière ou la face frontale proximale, et un déplacement dans cette direction sera désigné par "reculer".

Selon l'invention le manchon tubulaire présente une fente longitudinale comme est definit dans la revendication 1. Ce mode de réalisation permet de simplifier la fabrication et l'assemblage du dispositif selon l'invention.

Selon une variante de l'invention la zone élargie est prolongée par une languette comme est definit dans la revendication 4.

Selon une variante préférable, la fente présente en avant de la position initiale du porte-aiguille un rétrécissement pour le positionnement de l'ergot.

L'invention sera mieux comprise à la lecture de la description qui suit, faisant référence aux dessins annexés où :
- la figure 1 représente une vue en coupe longitudinale d'une première variante de réalisation du dispositif de sécurité ;
- la figure 2 représente une vue transversale selon AA ;
- les figures 3 à 5 représentent une vue de coupe, à une échelle réduite, du dispositif à trois étapes successives de mise en oeuvre ;
- la figure 6 représente une vue de coté d'une réalisation selon l'invention
- la figure 7 représente une vue agrandie d'un dispositif selon un deuxième mode de réalisation.

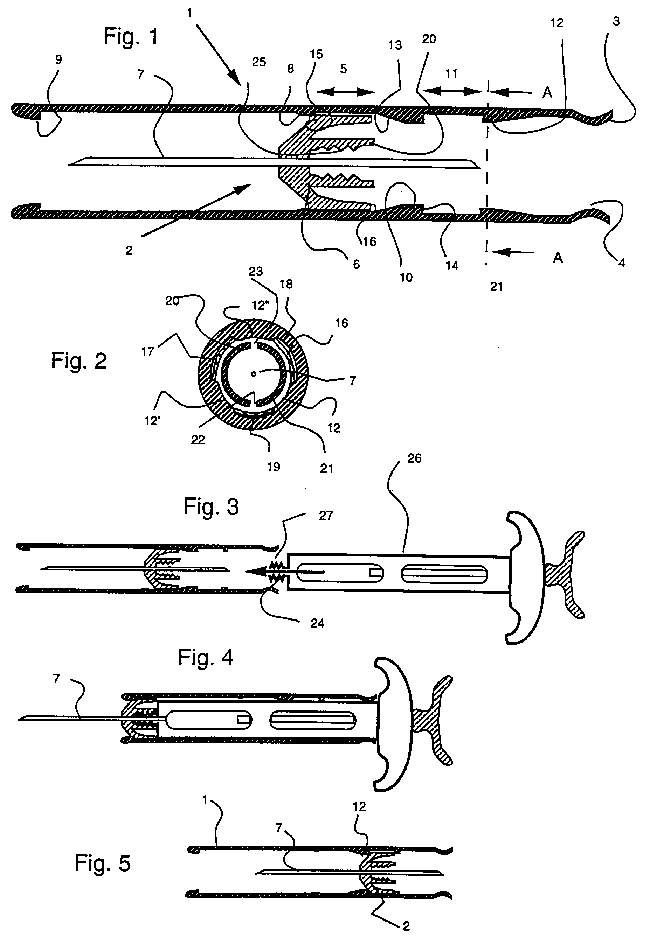

La figure 1 représente une vue schématique du dispositif de protection.

Il est constitué par un manchon (1) et par un porte-aiguille (2) mobile longitudinalement.

Le manchon (1) est constitué par une pièce tubulaire moulée en matière plastique, par exemple en polycarbonate transparent ou en polyéthylène.

La section intérieure du manchon (1) est déterminée de façon à permettre la mise en place sur le corps d'une seringue standard. Il présente à son extrémité arrière un tronçon conique (3) facilitant l'introduction du corps de la seringue. Une couronne annulaire (4) formant saillie par rapport à la surface intérieure du manchon supprime le jeu latéral de la seringue après assemblage. L'élasticité du manchon tubulaire permet une déformation radiale de la paroi pendant l'introduction du corps de la seringue et un blocage efficace pendant l'utilisation.

Le porte-aiguille (2) est constitué par une bague (6) en matière plastique, par exemple en polycarbonate ou en polyéthylène percé par un alésage central pour le passage de l'aiguille (7). La solidarisation de l'aiguille est réalisée par collage ou par tout autre moyen connu.

Le manchon (1) présente une collerette annulaire (8) assurant le positionnement initial du porte-aiguille (2) dans une zone initiale (5) dans laquelle l'aiguille (7) est intégralement protégée par la paroi latérale du manchon tubulaire (1). L'épaisseur de la collerette (8) est déterminée de manière à permettre le passage du tiroir du moule lors du moulage, ou du porte-aiguille (2) par déformation élastique de la paroi latérale, lors qu'une force axiale réduite est exercée sur respectivement le tiroir du moule ou le porte-aiguille.

Une variante consiste à mettre en oeuvre non pas une collerette, mais au moins un cran saillant s'étendant sur au moins un secteur angulaire limité, par exemple sur au moins un secteur d'une dizaine de degrés.

La partie avant du manchon tubulaire (1) présente une butée (9) constituée par une nervure annulaire réduisant la section intérieure du manchon (1) de manière à empêcher la sortie du porte-aiguille (2).

Une variante consiste à prévoir une paroi frontale présentant une surface intérieure complémentaire à l'extrémité distale du porte-aiguille (2), cette paroi frontale étant percée par un orifice pour le passage de l'aiguille et de la partie pointue du porte-aiguille. Cette solution est possible lorsque le tiroir du moule peut être retiré par l'extrémité opposée.

Le manchon tubulaire (1) présente également en arrière de la position initiale du porte-aiguille (2) des crans de blocage (10) définissant avec des crans protubérants (12) une zone de blocage inviolable (11) empêchant le déplacement du porte-aiguille (2) après utilisation. Les crans (12) se terminent du coté de la seringue par une rampe facilitant le centrage de la seringue.

Les crans de blocage (10) présentent une rampe distale (13) assurant une diminution progressive de la section intérieure du manchon tubulaire (1). Ce rétrécissement progressif permet le passage du porte-aiguille (2) dans la zone de blocage (11) par déformation élastique du manchon (1) ou des parois latérales du porte-aiguille. La déformation élastique du manchon (1) peut être obtenue par la présence d'une ou de plusieurs fentes longitudinales.

La surface arrière des crans de blocage (10) forme un épaulement (14) présentant une surface de blocage dans le plan transversal, perpendiculaire à l'axe du manchon (1). Cet épaulement assure le blocage du porte-aiguille par coopération avec une surface annulaire complémentaire (15) prévue sur la face avant du porte-aiguille.

Il est bien entendu possible d'autre forme d'ancrage, notamment en prévoyant des surfaces de contacts délimitant une rainure périphérique.

Pour faciliter le démoulage, les crans de blocage (10, 12) s'étendent sur des secteurs angulaires réduits, par exemple 60°, séparés par des zones cylindriques (17 à 19) s'étendant sur des secteurs angulaires intercalaires, comme représenté en figure 2.

Ce mode de réalisation permet de faciliter le démoulage du manchon par une rotation de 60° avant retrait du tiroir du moule.

Le porte-aiguille présente une paroi tubulaire (16) mince déformable élastiquement, afin de permettre le passage des crans (10) lorsqu'il est tiré en arrière par le corps de la seringue.

Il présente en outre deux mâchoires (20, 21) séparés par des fentes radiales (22, 23). Ces mâchoires présentent une section intérieure complémentaire de la section du col (24) habituellement prévu à l'extrémité frontale du corps de la seringue. Il est bien entendu possible de prévoir un nombre plus élevé de fentes.

La surface intérieure des mâchoires (20, 21) présente des crantages périphériques (25) en forme de pas de vis assurant la solidarisation temporaire sur le col (24) de la seringue.

Le fonctionnement du dispositif est le suivant: Le praticien déconditionne le dispositif et prépare la seringue (26) en introduisant dans le corps de la seringue une carpule contenant le liquide à injecter. Cette carpule présente une extrémité perforable disposée en arrière du col (24) du corps de la seringue (26). La seringue est présentée comme représentée en figure 3 dans l'axe du manchon tubulaire, et est introduite par un déplacement axial dans le manchon tubulaire (1) du dispositif.

L'ouverture conique (27) facilite l'introduction du corps de la seringue. Lorsque la partie frontale du corps de seringue vient en contact avec le porte-aiguille, la progression de la seringue par rapport au manchon (1) entraîne le déplacement du porte-aiguille (2) au-delà de la collerette (8), jusqu'à ce qu'il vienne en contact avec la butée (9). Le blocage par la butée (9) provoque l'encliquetage du col (24) du corps de seringue avec les mâchoires (20, 21).

La seringue est alors prête à l'emploi, comme représenté en figure 4. L'aiguille (7) est en position habituelle, et le corps de la seringue est emboîté dans le manchon tubulaire pour former un instrument chirurgical facile à manipuler.

En cours d'utilisation, le praticien peut procéder au retrait partiel du corps de seringue, en limitant la course relative du corps de seringue par rapport au manchon tubulaire (1) de manière à éviter que le porte-aiguille ne dépasse la zone initiale (5). Ce mouvement lui permet par exemple de protéger temporairement l'aiguille pour le remplacement de la carpule.

Après utilisation, le praticien retire la seringue du dispositif. Le clipsage du porte-aiguille sur le col (24) solidarise suffisamment le porte-aiguille (2) avec le corps de la seringue de manière à entraîner le porte-aiguille jusque dans la zone de blocage inviolable (11). A ce moment, la paroi tubulaire (16) du porte-aiguille (2) vient en butée contre les crans (12). La force exercée sur la seringue provoque le désaccouplement du porte-aiguille (2) et du col (24). Le porte-aiguille se trouve ainsi définitivement bloqué dans la zone de blocage (11), comme représenté en figure 5.

En cas de réintroduction de la seringue dans le dispositif de sécurité, la progression est bloquée par le porte-aiguille (2) en butée dans la zone de blocage (11). L'épaulement (14) des crans (10) forme avec la surface de contact annulaire (15) du porte-aiguille un ensemble résistant à des pressions axiales importantes, de même que les crans (12) pour des pressions axiales inverses.

Il est donc quasiment impossible de faire revenir l'aiguille (7) en position d'utilisation.

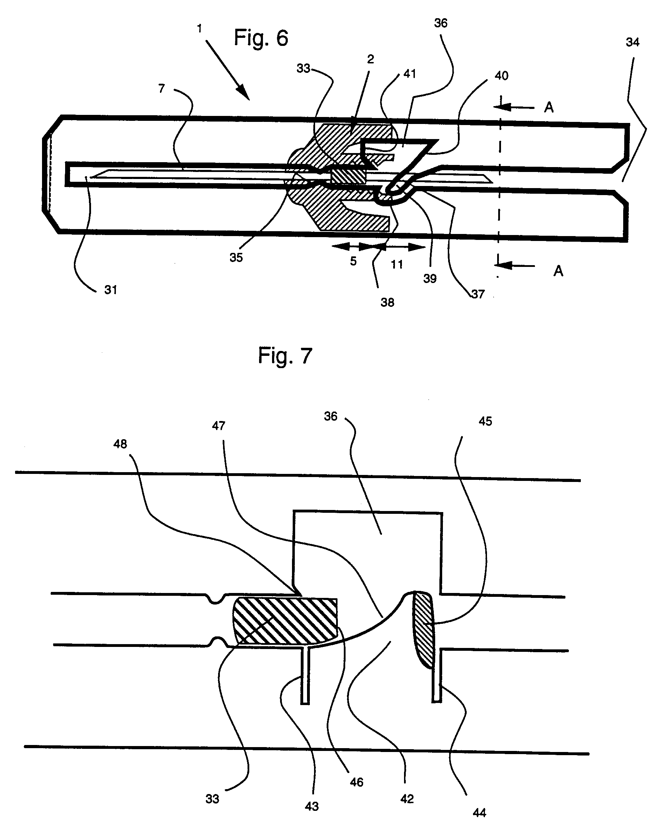

La figure 6 représente une variante de réalisation du dispositif de sécurité.

Le manchon tubulaire (1) est un tube partiellement fendu ouvert à son extrémité distale. Le bord de l'ouverture distale est conique et permet le blocage du porte-aiguille lorsque celui-ci est en position avancée.

Il présente une fente (31) s'étendant sur la paroi latérale depuis l'extrémité frontale (34) ouverte pour l'introduction du corps de la seringue, jusqu'à une région distale.

Le porte-aiguille (2) vue par transparence à travers la paroi du manchon tubulaire (1) présente un ergot latéral (33) saillant d'une largeur correspondant sensiblement à la largeur de la fente (31). La position initiale du porte-aiguille (2) est déterminée par une zone rétrécie (35) de la fente (31). Il sera maintenu vers l'arrière par la languette.

La zone de blocage inviolable (11) est déterminée par une zone élargie (36) de la fente (31). En arrière de cette zone de blocage (11) est prévue une languette (37) élastiquement déformable s'étendant suivant un axe formant avec l'axe longitudinal de la fente un angle compris entre 10 et 90 degrés, de préférence de l'ordre de 45°. Le bord opposé de la fente (31) présente un logement (38) de forme complémentaire à l'extrémité (39) de la languette (37). La dimension de ce logement (38) est inférieure aux dimensions de l'ergot (33) pour éviter que celui-ci ne vienne s'y loger lors de l'assemblage des composantes du dispositif.

L'ouverture de la zone élargie (36) est déterminée de façon à permettre le passage de l'ergot saillant (33).

Le fonctionnement de cette variante est le suivant :

L'assemblage du porte-aiguille (2) dans le manchon (1) s'effectue en positionnant le porte-aiguille dans l'axe du manchon (1), l'ergot étant dirigé de manière à pouvoir être enfilé dans la fente (31). On repousse le porte-aiguille jusqu'à ce que l'ergot (33) vienne en contact avec le bord arrière de la languette (37). En continuant à exercer une pression axiale sur le porte-aiguille (37), l'ergot repousse latéralement la languette (37) qui vient s'effacer latéralement dans la zone élargie (36) et libère ainsi le passage de la fente (31). L'ergot vient ensuite en contact avec la zone rétrécie (35), et le porte-aiguille occupe alors sa position initiale.

Les fentes sont démoulables de l'intérieur par déplacement axial du tiroir du moule. Elles peuvent être recouvertes de plastique pour éviter la déformation du manchon. Au démoulage, le tiroir du moule muni d'une partie protubérante pour la formation de la zone élargie (36) est retiré par déformation élastique du manchon.

L'introduction du corps de seringue dans le manchon tubulaire provoque comme pour la variante précédemment décrite l'avancement du porte-aiguille (2) jusqu'à ce que sa face avant vienne en butée l'extrémité avant du manchon (1). Le col du corps de la seringue vient alors s'encliqueter entre les mâchoires du porte-aiguille (2). Après utilisation, la seringue est retirée du dispositif de sécurité. Le col de la seringue entraîne le porte-aiguille vers l'arrière du manchon tubulaire. Lorsque le bord arrière de l'ergot (33) entre en contact avec le bord avant de la languette oblique (37), la traction exercée sur le porte-aiguille provoque une légère rotation du porte-aiguille (2) entraîne le déplacement latéral de l'ergot (33) dans la zone élargie (36). L'ergot vient ensuite en butée contre le bord arrière (40) de la zone élargie (36), et le col de la seringue se désaccouple du porte-aiguille.

L'ergot se trouve alors prisonnier de la zone élargie (36). Si l'on exerce à nouveau une pression sur l'arrière du porte-aiguille (2), l'ergot vient buter contre le bord avant (41) de la zone élargie (36). Ce bord avant (41) est orienté sensiblement perpendiculairement à l'axe longitudinal, voire légèrement oblique pour repousser l'ergot (33) vers la direction opposée à la fente (31) lorsqu'il progresse vers l'avant.

La figure 7 représente une vue agrandie d'un dispositif selon un troisième mode de réalisation.

La fente longitudinale (31) présente une zone élargie (36) dont les dimensions sont légèrement supérieures à la dimension de l'ergot (33). Le manchon présente par ailleurs une languette (42) s'étendant en face de la zone élargie (36) en travers de la fente longitudinale (31). Le manchon présente en avant et en arrière de la languette (42) des fentes radiales (43, 44) conférant une certaine souplesse à la languette (42), qui peut être repoussée élastiquement pour laisser le passage de l'ergot (33) lors de l'assemblage du porte-aiguille et du manchon (1). La languette (42) présente un bord d'attaque arrière (45) en biseau pour faciliter l'écartement de la languette (42) lors du passage de l'ergot (33).

Le bord arrière (46) de l'ergot (33) présente une forme complémentaire au bord avant (47) de la languette (42), afin de repousser l'ergot (33) dans la zone élargie (36) lors du retrait du porte-aiguille.

La zone élargie présente une protubérance (48) dirigée vers l'arrière du porte-aiguille et formant un obstacle au retrait de l'ergot (33) lorsqu'il est en position de blocage.

## Revendications

1. Dispositif de sécurité pour seringue à usage dentaire, constitué par un manchon tubulaire à l'intérieur duquel est prévu un porte-aiguille (2) mobile axialement entre une position avancée dans laquelle l'aiguille (7) dépasse l'extrémité distale du manchon tubulaire (1) et une position arrière dans laquelle l'aiguille (7) est entièrement rentrée dans le manchon tubulaire (1), le manchon tubulaire (1) présentant une section intérieure correspondant sensiblement à la section extérieure du corps de la seringue (26), le manchon (1) présentant des moyens (30) pour limiter le déplacement du porte-aiguille (2) en direction avant et à sa partie arrière un second moyen de blocage (36) permettant le passage du porte-aiguille (2) en direction arrière et empêchant le retour du porte-aiguille (2) en position avancée, caractérisé en ce que le manchon tubulaire (1) présente une fente longitudinale (31) s'étendant depuis une région distale (32) dudit manchon jusqu'à l'extrémité arrière (34), ladite fente comprenant une zone élargie (36) prolongée par une languette (37) déformable élastiquement s'étendant au repos à travers de la fente (31) et dont l'extrémité (39) est dirigée vers l'avant du manchon (1), et en ce que le porte-aiguille (2) présente un ergot (33) formant une saillie latérale susceptible de coulisser dans la fente (31).

2. Dispositif de sécurité pour seringue (26) à usage dentaire selon la revendication 1 caractérisé en ce que le bord de la fente (31) présente en face de la zone élargie (36) un logement (38) pour l'extrémité (39) de la languette (37).

3. Dispositif de sécurité pour seringue (26) à usage dentaire selon l'une quelconque des revendications 1 à 2 caractérisé en ce que la fente (31) présente en avant de la position initiale du porte-aiguille (2) un rétrécissement (35) pour le positionnement initial de l'ergot (33).

4. Dispositif de sécurité pour seringue à usage dentaire, constitué par un manchon tubulaire à l'intérieur duquel est prévu un porte-aiguille (2) mobile axialement entre une position avancée dans laquelle l'aiguille (7) dépasse l'extrémité distale du manchon tubulaire (1) et une position arrière dans laquelle l'aiguille (7) est entièrement rentrée dans le manchon tubulaire (1), le manchon tubulaire (1) présentant une section intérieure correspondant sensiblement à la section extérieure du corps de la seringue (26), le manchon (1) présente des moyens (9, 30), pour limiter le déplacement du porte-aiguille (2) en direction avant et à sa partie arrière un second moyen de blocage (10, 36) permettant le passage du porte-aiguille (2) en direction arrière et empêchant le retour du porte aiguille (2) en positon avancée, caractérisé en ce que le manchon tubulaire (1) présente une fente longitudinale (31) s'étendant depuis une région distale (32) dudit manchon jusqu'à l'extrémité arrière (34), ladite fente longitudinale (31) présentant une zone élargie (36) dont les dimensions sont légèrement supérieures à la dimension de l'ergot (33), le manchon présentant en outre une languette (42) s'étendant en face de la zone élargie (36) en travers de la vente longitudinale (31) délimité en avant et en arrière par des fentes radiales (43, 44).

## Claims

1. Safety device for a dental syringe, composed of a tubular sleeve inside which there is a needle holder (2) free to move axially between a forward position in which the needle (7) projects beyond the distal end of the tubular sleeve (1) and a rear position in which the needle (7) is fully retracted in the tubular sleeve (1), the tubular sleeve (1) having an inside cross-section corresponding approximately to the outside cross-section of the syringe body (26), the sleeve (1) having means (30) for limiting the movement of the needle holder (2) in the forwards direction, and a second blocking means (36) in its rear part allowing the needle holder (2) to pass in the backwards direction and preventing the needle holder (2) from returning to the forward position, characterized in that the tubular sleeve (1) has a longitudinal slit (31) extending from a distal region (32) of said sleeve as far as the rear end (34), said slit comprising a widened area (36) prolonged by an elastically deformable tab (37) extending at rest through the slit (31) and the end (39) of which is directed towards the front of the sleeve (1) and in that the needle holder (2) is fitted with a stud (33) forming a lateral projection capable of sliding in the slit (31).

2. Safety device for a dental syringe (26) according to claim 1, characterized in that the edge of the slit (31) has a housing (38) for the end (39) of the tab (37) in front of the widened area (36).

3. Safety device for a dental syringe (26) according to any one of claims 1 to 2, characterized in that the slit (31) has a narrowing (35) for the initial position of the stud (33) in front of the initial position of the needle holder (2).

4. Safety device for a dental syringe, composed of a tubular sleeve inside which there is a needle holder (2) free to move axially between a forward position in which the needle (7) projects beyond the distal end of the tubular sleeve (1) and a rear position in which the needle (7) is fully retracted in the tubular sleeve (1), the tubular sleeve (1) having an inside cross-section corresponding approximately to the outside cross-section of the syringe body (26), the sleeve (1) having means (9, 30) for limiting the movement of the needle holder (2) in the forward direction, and a second blocking means (10, 36) in its rear part allowing the needle holder (2) to pass in the reverse direction and preventing the needle holder (2) from returning to the forward position, characterized in that the tubular sleeve (1) has a longitudinal slit (31) extending from a distal region (32) of said sleeve as far as the rear end (34), said longitudinal slit (31) comprising a widened area (36), the dimensions of which are slightly wider than the dimension of the stud (33), the sleeve also has a tab (42) extending in front of the widened area (36) through the longitudinal slit (31) delimited at the front and back by radial slits (43, 44).

## Patentansprüche

1. Sicherheitsvorrichtung für eine zahnärztliche Spritze, bestehend aus einer rohrförmigen Hülse, in der ein Nadelträger (2) vorgesehen ist, der zwischen einer vorgeschobenen Position, in der die Nadel (7) das entfernte Ende der rohrförmigen Hülse (1) überschreitet, und einer hinteren Position, in der die Nadel (7) völlig in die rohrförmige Hülse (1) eingeschoben ist, axial beweglich ist, wobei die rohrförmige Hülse (1) einen Innenquerschnitt aufweist, der in etwa dem Aussenquerschnitt des Spritzenkörpers (26) entspricht, wobei die Hülse (1) Mittel (30) aufweist, um das Verschieben des Nadelträgers (2) nach vorne zu begrenzen, und an ihrem hinteren Teil ein zweites Blockiermittel (36), das den Übergang des Nadelträgers (2) nach hinten ermöglicht und die Rückkehr des Nadelträgers (2) in die vorgeschobene Position verhindert, dadurch gekennzeichnet, dass die rohrförmige Hülse (1) einen länglichen Schlitz (31) aufweist, der sich aus einer entfernten Region (32) der besagten Hülse bis zum hinteren Ende (34) erstreckt, wobei der Schlitz eine verbreiterte Zone (36) umfasst, die von einer elastisch verformbaren Lasche (37) verlängert wird, die in Ruhestellung den Schlitz (31) durchquert und deren Ende (39) zur Vorderseite der Hülse (1) hin gerichtet ist, und dass der Nadelträger (2) einen Nocken (33) aufweist, der einen seitlichen Vorsprung bildet, der im Schlitz (31) gleiten kann.

2. Sicherheitsvorrichtung für eine zahnärztliche Spritze (26) nach Anspruch 1, dadurch gekennzeichnet, dass der Rand des Schlitzes (31) gegenüber der verbreiterten Zone (36) eine Aufnahme (38) für das Ende (39) der Lasche (37) aufweist.

3. Sicherheitsvorrichtung für eine zahnärztliche Spritze (26) nach einem der Ansprüche 1 bis 2, dadurch gekennzeichnet, dass der Schlitz (31) vor der ursprünglichen Position des Nadelträgers (2) eine Verengung (35) für die ursprüngliche Positionierung des Nockens (33) aufweist.

4. Sicherheitsvorrichtung für eine zahnärztliche Spritze, bestehend aus einer rohrförmigen Hülse, in der ein Nadelträger (2) vorgesehen ist, der zwischen einer vorgeschobenen Position, in der die Nadel (7) das entfernte Ende der rohrförmigen Hülse (1) überschreitet, und einer hinteren Position, in der die Nadel (7) völlig in die rohrförmige Hülse (1) eingeschoben ist, axial beweglich ist, wobei die rohrförmige Hülse (1) einen Innenquerschnitt aufweist, der in etwa dem Aussenquerschnitt des Spritzenkörpers (26) entspricht, wobei die Hülse (1) Mittel (9, 30) aufweist, um das Verschieben des Nadelträgers (2) nach vorne zu begrenzen, und an ihrem hinteren Teil ein zweites Blockiermittel (10, 36), das den Übergang des Nadelträgers (2) nach hinten ermöglicht und die Rückkehr des Nadelträgers (2) in die vorgeschobene Position verhindert, dadurch gekennzeichnet, dass die rohrförmige Hülse (1) einen länglichen Schlitz (31) aufweist, der sich aus einer entfernten Region (32) der besagten Hülse bis zum hinteren Ende (34) erstreckt, wobei der besagte längliche Schlitz (31) eine verbreiterte Zone (36) umfasst, deren Abmessungen etwas grösser sind als diejenige des Nockens (33), wobei die Hülse weiters eine Lasche (42) aufweist, die sich gegenüber der verbreiterten Zone (36) durch den länglichen Schlitz (31) erstreckt, vorne und hinten durch radiale Schlitze (43, 44) begrenzt.
